(19) Europäisches Patentamt
European Patent Office
Office européen des brevets

(11) Publication number: **0 345 998 A1**

(12) # EUROPEAN PATENT APPLICATION

(21) Application number: **89305513.7**

(51) Int. Cl.⁴: **C07D 205/08 , C07D 403/06**

(22) Date of filing: **01.06.89**

(30) Priority: **06.06.88 US 202289**

(43) Date of publication of application: **13.12.89 Bulletin 89/50**

(84) Designated Contracting States: **AT BE CH DE ES FR GB GR IT LI NL SE**

(71) Applicant: **ELI LILLY AND COMPANY**
**Lilly Corporate Center**
**Indianapolis Indiana 46285(US)**

(72) Inventor: **Doecke, Christopher William**
**550 San Ricardo Drive**
**Greenwood Indiana 46142(US)**
Inventor: **Hoying, Richard Charles**
**5514 Manker Street**
**Indianapolis Indiana 46227(US)**

(74) Representative: **Tapping, Kenneth George et al**
**Erl Wood Manor**
**Windlesham Surrey, GU20 6PH(GB)**

(54) **Process and intermediates for beta-lactam antibiotics.**

(57) The present invention provides a process for preparing β-keto ester intermediates to 1-carba(1-dethia)-3-cephem-4-carboxylic acids, and intermediates thereof.

EP 0 345 998 A1

# PROCESS AND INTERMEDIATES FOR β-LACTAM ANTIBIOTICS

This invention relates to the synthesis of intermediates to 1-carba(1-dethia)-3-cephem-4-carboxylic acids and derivatives thereof. The process of the invention is preferably used to prepare intermediates to a class of potent orally active 1-carba(1-dethia)-3-cephem-4-carboxylic acid antibiotics described by J. Hashimoto et al. in U.S. Patent Nos. 4,335,211 and 4,708,956.

The 1-carba(1-dethia)-3-cephem-4-carboxylic acids, hereinafter 1-carbacephalosporins, possess the 4,6 bicyclic ring system represented by the following structural formula

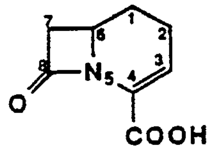

wherein the arbitrary numbering system employed according to the cephem nomenclature system is used as indicated.

The 1-carbacephalosporins thus far have not been obtained from natural sources, for example, as microbial metabolites. Accordingly, methods for the total synthesis of these promising compounds are highly desirable, particularly methods which are adaptable to large scale manufacture.

Evans et al. in U.S. Patent No. 4,665,171 disclose the synthesis of a β-keto ester $C_1$-$C_4$ alkyl 5-[3β-(protected amino)azetidin-2-one-4β-yl]-3-oxo-pentanoate by subjecting the corresponding 3β-(protected amino)-4β-[2-(5-alkoxycyclohex-1,4-dienyl)ethyl]azetidinone to ozonolysis. This procedure is laborious, relatively low yielding and generally requires purification of the desired compound by column chromatography. The process disclosed in the patent is not desirable for the synthesis of the compounds on a large scale.

The present invention provides a process for preparing intermediates to 1-carbacephalosporins which is particularly well suited for a large scale industrial setting. More specifically, the present invention relates to a process for preparing a β-keto ester of the Formula I

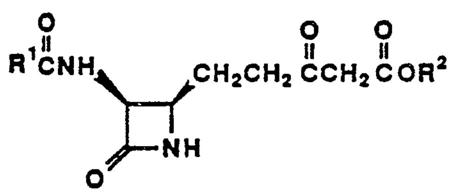

I

wherein $R^1$ is $C_1$-$C_6$ alkyl; a phenyl group

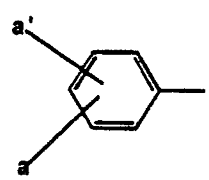

wherein a and a′ independently are hydrogen, $C_1$-$C_4$ alkyl, $C_1$-$C_4$ alkoxy or halogen; a group represented by the formula

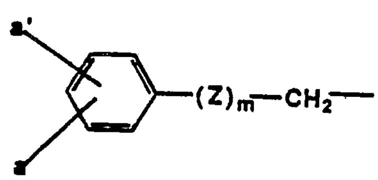

wherein Z is O or S, m is 0 or 1, and a and a′ have the same meanings as defined above; or $R^1$ is $R^{1'}O$ wherein $R^{1'}$ represents $C_1$-$C_4$ alkyl, $C_5$-$C_7$ cycloalkyl, benzyl, nitrobenzyl, methoxybenzyl, or halobenzyl; and $R^2$ is $C_1$-$C_4$ alkyl, allyl, 2,2,2-trichloroethyl, 2,2,2-tribromoethyl, β-tri($C_1$-$C_4$ alkyl)silylether, benzyl, $C_1$-$C_4$ alkylbenzyl, $C_1$-$C_4$ alkoxybenzyl, nitrobenzyl, or chlorobenzyl, comprising reacting a compound of the Formula II

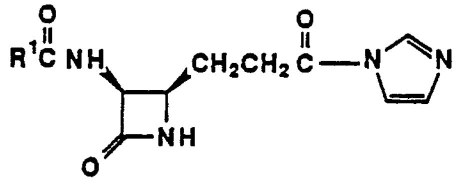

II

wherein $R^1$ is as defined above, with a propanedioic acid mono(substituted) ester hemimagnesium salt of the Formula III

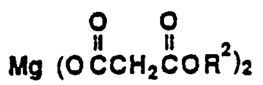

III

wherein $R^2$ is as defined above, in a suitable solvent.

The present invention also provides a compound of the Formula II

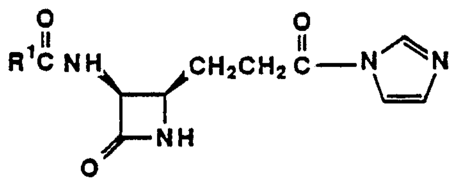

II

wherein $R^1$ is as defined above.

All temperatures stated herein are in degrees Celsius. All quantities specified are in weight units, except for liquids, which are in volume units.

The term "$C_1$-$C_6$ alkyl" represents a straight or branched alkyl chain having from 1 to 6 carbon atoms. Typical $C_1$-$C_6$ alkyl groups include methyl, ethyl, n-propyl, isopropyl, n-butyl, sec.-butyl, t-butyl, n-pentyl, isopentyl, n-hexyl, isohexyl and the like.

Halogen represents fluoro, chloro, bromo or iodo.

$C_1$-$C_4$ Alkoxy represents a straight or branched alkoxy chain having from 1 to 4 carbon atoms. Typical $C_1$-$C_4$ alkoxy groups include methoxy, ethoxy, n-propoxy, isopropoxy, n-butoxy, isobutoxy and the like.

$C_5$-$C_7$ Cycloalkyl represents cyclopentyl, cyclohexyl or cycloheptyl.

While the entire scope of process variables taught herein are believed operable, the present process does have preferred aspects. In the above formulas, $R^2$ is preferable nitrobenzyl, and especially 4-nitrobenzyl. $R^1$ is preferably a group represented by the formula

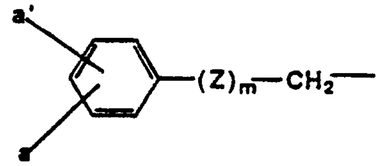

wherein a and a′ are hydrogen, m is 1 and Z is O. Other preferred aspects of the present invention will be noted hereinafter.

The process of the present invention is carried out as follows. At least one equivalent in relation to the amount of a compound of Formula II, and preferably an excess, of a propanedioic acid mono(substituted)

ester hemimagnesium salt of Formula III is combined with a compound of Formula II in a suitable solvent. Preferably, a solution of the compound of Formula III in a suitable solvent is added dropwise to a slurry of a compound of Formula II in a suitable solvent. Suitable solvents will be inert to the reaction conditions and include the halogenated hydrocarbon solvents such as methylene chloride, 1,2-dichloroethane, chloroform, carbon tetrachloride, 1,1,1-trichloroethane, 1,1,2,2-tetrachloroethane, pentachloroethane and the like; the hydrocarbon solvents such as hexane, pentane and the like; the ethers such as diethyl ether, and the cyclic ethers such as tetrahydrofuran (THF). Of these, the cyclic ethers are preferred, and THF is especially preferred.

As in any chemical process the concentration of reactants in the reaction mixture is not critical, but it is preferred to employ the least amount of solvent necessary to keep the reactants in solution. The range of practical concentration depends in part on the power of the mixing equipment used, and in part on the solubility of the reactants in the chosen solvent. Generally it is convenient to conduct the reaction at a concentration in the range of about 500 ml to 1L of halogenated hydrocarbon solvent per gram mole of a compound of Formula II, with about 500 ml per gram mole being preferred. This concentration range is both economical and convenient in large scale production. Higher concentrations of reactants are feasible with the understanding that it will not be possible to carry out the desired smooth conversion of reactants from step to step if the starting materials are allowed to partially precipitate out of solution during the reaction period.

The process of the present invention is substantially complete after about 10 minutes to 24 hours or more when conducted at a temperature in the range of about -25 °C to about 100 °C. The reaction is preferable conducted for approximately 4 hours at a temperature in the range of about 25 °C to about 40 °C.

The product of the present invention may be isolated by standard conditions. Typically, a water immiscible organic solvent such as methylene chloride may be added and the resulting organic phase washed with aqueous acid, such as hydrochloric acid, followed by aqueous base, such as sodium bicarbonate. The resulting solution is then dried and concentrated, typically under vacuum, to provide the desired β-keto ester of Formula I. Preferably the water immiscible organic solvent is not added, and the precipitated solid β-keto ester collected by standard techniques such as vacuum filtration.

The compounds prepared by the present process may be further purified if desired by standard techniques such as crystallization from common solvents or column chromatography over solid supports such as silica gel or alumina.

The process of the present invention is particularly advantageous because it is capable of preparing the valuable 1-carbacephalosporin intermediates which are its products in high purity without additional purification. Further, the process may be carried out under simplified conditions in an inexpensive manner, and as such the process is particularly well suited for the large scale industrial synthesis of the desired intermediate to the 1-carbacephalosporin product. Finally, since the product of the present process may be isolated without the use of expensive and laborious column chromatography procedures, the reaction is economically advantageous. This is especially important when using the present process in a large scale operation.

As noted above the compounds prepared by the process of the present invention are useful as intermediates in the synthesis of 1-carbacephalosporins. For example, the β-keto ester of Formula I is reacted with an arylsulfonyl azide to provide the α-diazo-β-keto ester which undergoes rhodium catalyzed cyclization to the corresponding 3-hydroxy-1-carbacephalosporin as taught by Evans et al. in U.S. Patent No. 4,665,171, herein incorporated by reference.

The starting propanedioic acid mono(substituted)ester hemimagnesium salts of Formula III employed in the present process are known compounds prepared by prior art procedures. The starting material of Formula II is provided as yet another embodiment of the present invention and is prepared by standard procedures. Preferably, a cis-4-oxo-3-(substituted)amino-2-azetidinepropanoic acid is combined with at least one equivalent of carbonyldiimidazole typically in THF under standard conditions to afford the appropriate compound of Formula II.

The cis-4-oxo-3-(substituted)amino-2-azetidinepropanoic acid may be prepared as follows. First, phthalimidoacetyl chloride is reacted with the enamine formed with anisidine and cinnamaldehyde to provide via cycloaddition the 1-(4-methoxyphenyl)-3β-phthalimido-4-(2-phenylvinyl)azetidin-2-one represented by the following structural formula

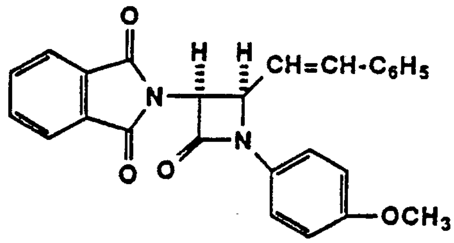

The phthaloyl group is removed from the cyclocondensation product with methylhydrazine to provide the corresponding 3β-aminoazetidinone. The latter can then be acylated by standard coupling methods to provide the β-acylamino-4-(2-carboxyvinyl)azetidin-2-one having the 4-methoxyphenyl group in the 1-position. The 4-methoxyphenyl group is then removed by known procedures with ceric ammonium nitrate to provide the 1H azetidinone represented by the following formula

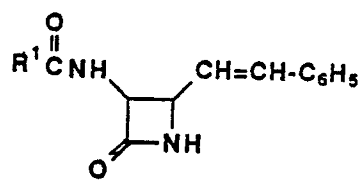

In the above formula, $R^1$ has the same meaning as defined above.

The azetidinone is then reacted with ozone in an inert solvent, for example, a mixture of methyl alcohol and methylene chloride to provide the desired 4-formylazetidinone represented by the following structural formula

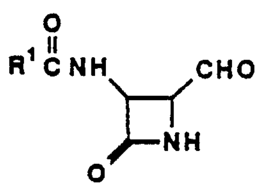

wherein $R^1$ has the same meanings as defined above.

The compound wherein an esterified 2-carboxyvinyl group exists at the 4-position is prepared with the 4-formylazetidinone and an ester of a di-($C_1$-$C_4$ alkyl)phosphononoacetic acid, for example, benzyl diethylphosphonoacetate. The reaction is carried out in an inert solvent such as acetonitrile in the presence of a tertiary amine, preferably a hindered tertiary amine such as diisopropylethylamine and a lithium halide, for example, lithium chloride. The reaction is carried out at room temperature and can be followed by thin layer chromatography on silica gel by employing ethyl acetate hexane for elution. The 2-esterified carboxyvinyl substituted azetidinone is obtained from the reaction mixture by conventional isolation procedures and is purified by chromatography over silica gel.

The compound thus prepared is converted by catalytic hydrogenation over palladium catalyst to the desired carboxylic acid starting material. During the hydrogenation the ester group is removed and the vinyl group of the 2-esterified carboxyvinyl substituent is reduced to provide the 2-carboxyethyl azetidinone. The hydrogenation is carried out in an inert solvent such as tetrahydrofuran or an alcohol such as methanol or ethanol or mixtures of such solvents at an elevated hydrogen pressure of between about 40 and about 100 psi. The palladium catalyst employed may be any of those commonly employed for hydrogenation such as palladium metal itself or a supported palladium catalyst such as 5 or 10% palladium on carbon, palladium on barium carbonate and the like. The hydrogenation proceeds satisfactorily at room temperature and can be followed by hydrogen absorption. Following the reduction, the catalyst is removed by filtration, the solvent evaporated and the residue containing the product dissolved in a water immiscible solvent such as ethyl acetate. The product which is the free carboxylic acid is extracted from the solution with a base such as sodium bicarbonate or sodium carbonate and, following acidification of the aqueous extract, the free acid is extracted from the aqueous phase with a water immiscible solvent, for example, methylene chloride. Evaporation of the extract affords the desired 2-carboxyethyl compound.

The following Examples further illustrate specific aspects of the present invention. The Examples are

not intended to be limiting to the scope of the process of the invention in any respect and should not be so construed.

## Example 1

cis-β,4-Dioxo-3-[(phenoxyacetyl)amino]-2-azetidinepentanoic acid, (4-nitrophenyl)methyl ester

A. Propanedioic acid mono[(4-nitrophenyl)methyl] ester hemimagnesium salt

To a solution of 9.9124 g (41.4 mmol) of 95% pure propanedioic acid mono[(4-nitrophenyl)methyl] ester dissolved in 35 ml of THF was added 2.36 g (20.7 mmol) of 95% pure magnesium ethoxide. The reaction mixture was stirred at about 40° C for about two hours to provide propanedioic acid mono[(4-nitrophenyl)-methyl] ester hemimagnesium salt which was used directly as described below.

B. cis-N-[2-[3-(1H-Imidazol-1-yl)-3-oxopropyl]-4-oxo-3-azetidinyl]-2-phenoxyacetamide

A mixture of 5.0034 g (17.1 mmol) of cis-4-oxo-3-[(phenoxyacetyl)amino]-2-azetidinepropanoic acid and 3.059 g (18.8 mmol) of carbonyldiimidazole in 35 ml of THF was stirred at 35° C for 90 minutes to provide a thick white slurry containing cis-N-[2-[3-(1H-imidazol-1-yl)-3-oxopropyl]-4-oxo-3-azetidinyl]-2-phenoxyacetamide. The reaction mixture was used directly in the following reaction.

C. The solution containing propanedioic acid mono[(4-nitrophenyl)methyl] ester hemimagnesium salt prepared as described above was added portionwise to the slurry containing cis-N-[2-[3-(1H-imidazol-1-yl)-3-oxopropyl]-4-oxo-3-azetidinyl]-2-phenoxyacetamide also prepared above over a period of about 15 minutes at 35° C. After addition the reaction mixture was nearly homogeneous. The reaction mixture was stirred at 35° C for an additional 30 minutes at which point a precipitate began to form. The reaction mixture was stirred for four hours at 35° C and 200 ml of methylene chloride was added. The organic layer was washed with 100 ml of 1N hydrochloric acid, 150 ml of a saturated sodium bicarbonate solution at pH 7, and 100 ml of a saturated sodium bicarbonate solution at pH 10. The organic phase was dried over anhydrous magnesium sulfate. The solution was triturated and concentrated under vacuum to provide 6.87 g of cis-β,4-dioxo-3-[(phenoxyacetyl)amino]-2-azetidinepentanoic acid, (4-nitrophenyl)methyl ester. Yield 85.6%. Optical Rotation 38.6°.

## Example 2

cis-β,4-Dioxo-3-[(phenoxyacetyl)amino]-2-azetidinepentanoic acid, (4-nitrophenyl)methyl ester

A. Propanedioic acid mono[(4-nitrophenyl)methyl] ester hemimagnesuim salt

To a solution of 29.7284 g (118 mmol) of 95% pure propanedioic acid mono[(4-nitrophenyl)methyl] ester dissolved in 100 ml of THF was added 7.092 g (59 mmol) of 95% pure magnesium ethoxide. The reaction mixture was stirred at about 40° C for one hour to provide propanedioic acid mono[(4-nitrophenyl)-methyl) ester hemimagnesuim salt. The mixture was cooled to room temperature and held for further processing as described below.

B. cis-N-[2-[3-(1H-imidazol-1-yl)-3-oxopropyl]-4-oxo-3-azetidinyl]-2-phenoxyacetamide

A mixture of 15.0059 g (51.3 mmol) of cis-4-oxo-3-[(phenoxyacetyl)amino]-2-azetidinepropanoic acid and 9.1634 g (56.5 mmol) of carbonyldiimidazole in 100 ml of THF was stirred at 30° C for 50 minutes. An

additional 100 ml of THF was added to the mixture, and the mixture containing cis-N-[2-[3-(1H-imidazol-1-yl)-3-oxopropyl]-4-oxo-3-azetidinyl]-2-phenoxyacetamide was used directly in the following reaction.

C. The solution containing propanedionic acid mono[(4-nitrophenyl)methyl] ester hemimagnesium salt prepared above was added to the slurry containing cis-N-[2-[3-(1H-imidazol-1-yl)-3-oxopropyl]-4-oxo-3-azetidinyl]-2-phenoxyacetamide dropwise over a period of 40 minutes while maintaining the temperature of the reaction mixture at about 30° C. The mixture was stirred for four hours at 30° C, cooled to 10° C, and 600 ml of 0.05 N HCL was added dropwise over a period of 35 minutes. The mixture was filtered and five hundred milliliters of water were added to the solid followed by 9.9 g of sodium bicarbonate. The resulting suspension was stirred for 10 minutes and the precipitated solid was collected by vacuum filtration. The solid was washed twice with 100 ml portions of water, once with isopropyl alcohol and once with hexanes. The resulting material was vacuum dried at 30° C to provide 16.98 g of cis-β,4-dioxo-3-[(phenoxyacetyl)-amino]-2-azetidinepentanoic acid, (4-nitrophenyl)methyl ester as a white solid. Yield 70.5%. Optical Rotation (DMSO) = +43.37.

## Example 3

cis-β,4-dioxo-3-[(phenoxyacetyl)amino]-2-azetidinepentanoic acid, (4-nitrophenyl)methyl ester

A. Propanedioic acid mono[(4-nitrophenyl)methyl]ester hemimagnesium salt

To a solution of 29.7203 g (118 mmol) of 95% pure propanedionic acid mono[(4-nitrophenyl)methyl] ester dissolved in 75 ml of THF was added 6.737 g (59 mmol) of magnesium ethoxide. The reaction mixture was stirred at 40° C for one hour and cooled to room temperature. The mixture contained propanedioic acid mono[(4-nitrophenyl)methyl]ester hemimagnesium salt and was used directly as described below.

B. cis-N-[2-[3-(1H-Imidazol-1-y)-3-oxopropyl]-4-oxo-3-azetidinyl]-2-phenoxyacetamide

A mixture of 15.0048 g (51.3 mmol) of cis-4-oxo-3-[(phenoxyacetyl)amino]-2-azetidinepropanoic acid and 9.1568 g (56.5 mmol) of carbonyldiimidazole in 125 ml of THF was stirred at 30° C for 45 minutes to provide a solution containing cis-N-[2-[3-(1H-imidazol-1-y)-3-oxopropyl]-4-oxo-3-azetidinyl]-2-phenoxyacetamide which was used directly in the following reaction.

C. The solution containing propanedionic acid mono[(4-nitrophenyl)methyl)ester hemimagnesium salt prepared as described above was added dropwise over a 35 minute period to the slurry containing cis-N-[2-[3-(1H-imidazol-1-yl)-3-oxopropyl]-4-oxo-3-azetidinyl]-2-phenoxyacetamide at 30° C. The resulting mixture was stirred at 30° C for three hours and 600 ml of 0.05 N HCl was added dropwise over a period of 45 minutes. The reaction mixture was cooled to 0° C for about one hour. The precipitated solid was collected by vacuum filtration was washed with chilled water. The solid was slurried in 200 ml of water and a solution of 9.9 g of sodium bicarbonate in 200 ml of water was added. The resulting slurry was stirred at about 5° C for 30 minutes and filtered. The collected solid was washed twice with 100 ml portions of water, once with 100 ml of isopropyl alcohol, twice with 100 ml portions of hexane and dried at 40° C in a vacuum oven to provide 19.03 g of cis-β,4-dioxo-3-[(phenoxyacetyl)amino]-2-azetidinepentanoic acid, (4-nitrophenyl)methyl ester. Yield 79%. Optical Rotation (DMSO) = +37.1.

## Example 4

cis-β,4-Dioxo-3-[(phenoxyacetyl)amino]-2-azetidinepentanoic acid, (4-nitrophenyl)methyl ester

A. Propanedioic acid mono[(4-nitrophenyl)methyl]ester hemimagnesium salt

To a solution of 39.6273 g (157.4 mmol) of propanedioic acid mono[(4-nitrophenyl)methyl]ester dissolved in 80 ml of dry THF was added 8.9822 g (78.7 mmol) of magnesium ethoxide. The resulting mixture was stirred at about 40 °C for about one hour to provide a solution containing propanedioic acid mono[(4-nitro phenyl)methyl] ester hemimagnesium salt. The solution was used directly as described below.

B. cis-N-[2-[3-(1H-Imidazol-1-yl)-3-oxopropyl]-4-oxo-3-azetidinyl]-2-phenoxyacetamide

A slurry of 20.0184 g (68.4 mmol) of cis-4-oxo-3-[(phenoxyacetyl)amino]-2-azetidinepropanoic acid and 12.2181 g (75.3 mmol) of carbonyldiimidazole in 170 ml of THF was heated at 30 °C for one hour to provide a slurry containing cis-N-[2-[3-(1H-imidazol-1-yl)-3-oxo-propyl]-4-oxo-3-azetidinyl]-2-phenoxyacetamide which was used directly in the following reaction.

C. The solution of propanedioic acid mono[(4-nitrophenyl)methyl] ester hemimagnesium salt prepared above was added dropwise over a 60 minute period to the slurry containing cis-N-[2-[3-(1H-imidazoly-1-yl)-3-oxopropyl]-4-oxo-3-azetidinyl]-2-phenoxyacetamide also prepared above while maintaining the temperature of the reaction mixture at 30 °C. The resulting mixture was stirred at 30 °C for four hours and a solution of 250 ml of water and 300 ml of 1N HCl was added dropwise over a period of 40 minutes. The suspension was cooled to 0 °C for several hours. The aqueous layer was decanted and the residue was slurried in 300 ml of water at 0 °C. A solution of 13.2 g of sodium bicarbonate in 300 ml of water was added and the resulting slurry was stirred at 0 °C for several hours and filtered. The collected solid was washed twice with 100 ml portions of water, once with 100 ml of isopropyl alcohol, twice with 100 ml portions of hexanes and dried in a vacuum oven at 35 °C to provide 26.0 g of cis-β,4-dioxo-3-[(phenoxyacetyl)amino]-2-azetidinepentanoic acid, (4-nitrophenyl)methyl ester. Yield 80.9%

## Claims

1. A process for preparing a β-keto ester of the Formula I

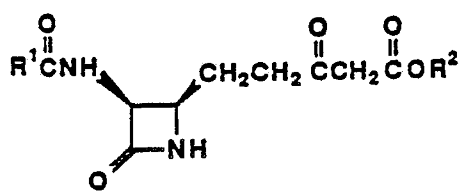

I

wherein $R^1$ is $C_1$-$C_6$ alkyl; a phenyl group

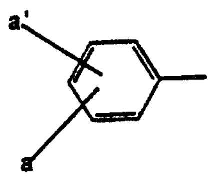

wherein a and a′ independently are hydrogen, $C_1$-$C_4$ alkyl, $C_1$-$C_4$ alkoxy or halogen; a group represented by the formula

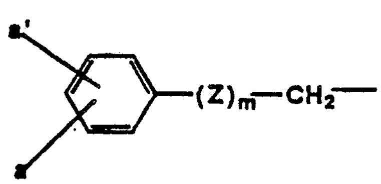

wherein Z is O or S, m is 0 or 1, and a and a′ have the same meanings as defined above; or $R^1$ is $R^{1'}O$

wherein $R^{1'}$ represents $C_1$-$C_4$ alkyl, $C_5$-$C_7$ cycloalkyl, benzyl, nitrobenzyl, methoxybenzyl, or halobenzyl; and $R^2$ is $C_1$-$C_4$ alkyl, allyl, 2,2,2-trichloroethyl, 2,2,2-tribromoethyl, $\beta$-tri($C_1$-$C_4$ alkyl)silylether, benzyl, $C_1$-$C_4$ alkylbenzyl, $C_1$-$C_4$ alkoxybenzyl, nitrobenzyl, or chlorobenzyl, comprising reacting a compound of the Formula II

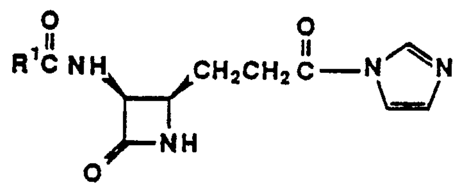

II

wherein $R^1$ is as defined above, with a propanedioic acid mono(substituted) ester hemimagnesium salt of the Formula III

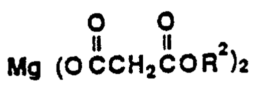

III

wherein $R^2$ is as defined above, in a suitable solvent.

2. A process of Claim 1 wherein $R^1$ is phenoxy methylene.

3. A process of Claim 2 wherein $R^2$ is (4-nitrophenyl)methyl.

4. A process of Claim 1 wherein the suitable solvent is tetrahydrofuran.

5. A compound of the Formula II

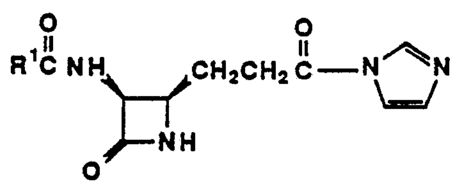

II

wherein $R^1$ is $C_1$-$C_6$ alkyl; a phenyl group

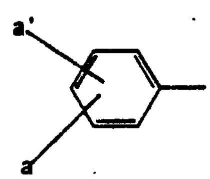

wherein a and a′ independently are hydrogen, $C_1$-$C_4$ alkyl, $C_1$-$C_4$ alkoxy or halogen; a group represented by the formula

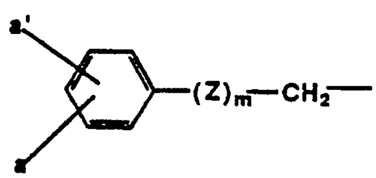

wherein Z is O or S, m is 0 or 1, and a and a′ have the same meanings as defined above; or $R^1$ is $R^{1'}O$ wherein $R^{1'}$ represents $C_1$-$C_4$ alkyl, $C_5$-$C_7$ cycloalkyl, benzyl, nitrobenzyl, methoxybenzyl, or halobenzyl.

6. A compound of Claim 5 wherein $R^1$ is phenoxy methylene.

European Patent Office

# EUROPEAN SEARCH REPORT

Application number

EP 89305513.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US - A - 4 665 171 (EVANS et al.) * Columns 7,10; example 5 * -- | 1-5 | C 07 D 205/08<br>C 07 D 403/06 |
| Y | JOURNAL OF THE AMERICAN CHEMICAL SOCIETY, vol. 102, no. 19, 1980 SALZMANN et al. "A stereo-controlled synthesis of (+)-thienamycin" pages 6161-6163 * Page 6162, scheme II; page 6163, left column: compounds 15,17 * ---- | 1-5 | |
| | | | TECHNICAL FIELDS SEARCHED (Int. Cl.4) |
| | | | C 07 D 205/00<br>C 07 D 403/00 |

The present search report has been drawn up for all claims

| Place of search | Date of completion of the search | Examiner |
|---|---|---|
| VIENNA | 29-08-1989 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82